# EUROPÄISCHE PATENTSCHRIFT

(11) **EP 0 998 675 B1**
(45) Veröffentlichungstag und Bekanntmachung des Hinweises auf die Patenterteilung: **17.03.2010**
(21) Anmeldenummer: 98942582.2
(22) Anmeldetag: 20.07.1998
(51) Int. Cl.: G01N 33/543, G01N 33/52, G01N 33/576, C12Q 1/68

(54) **VERWENDUNG VON KONTROLLFLÄCHEN ZUR DETEKTION VON STÖRPROBEN IN EINEM NACHWEISVERFAHREN**
USE OF CHECK SURFACES FOR IDENTIFYING DISTURBING SAMPLES IN A DETECTION PROCEDURE
UTILISATION DE SURFACES DE CONTROLE POUR DETECTER DES ECHANTILLONS PERTURBES DANS UN PROCEDE DE DETECTION

(30) Priorität: 22.07.1997 DE 19731465
(43) Veröffentlichungstag der Anmeldung: 10.05.2000
(73) Patentinhaber: Roche Diagnostics GmbH, 68305 Mannheim (DE)
(72) Erfinder: KARL, Johann, D-82380 Peissenberg (DE); LENZ, Helmut, D-82327 Tutzing (DE); KRAUSE, Friedemann, D-82377 Penzberg (DE); FINCKH, Peter, D-82211 Breitbrunn (DE); HORNAUER, Hans, D-82380 Peissenberg (DE); BERGER, Johann, A-8020 Graz (AT)
(74) Vertreter: Weiss, Wolfgang
(86) Internationale Anmeldenummer: PCT/EP1998/004533
(87) Internationale Veröffentlichungsnummer: WO 1999/005525

(56) Entgegenhaltungen:
- EP-A- 0 331 068
- WO-A-96/14338
- US-A- 4 558 013
- US-A- 5 160 701
- US-A- 5 356 785
- US-A- 5 705 353

## Beschreibung

Die Erfindung betrifft eine Festphase mit mindestens einer Testfläche zum Nachweis eines Analyten, welche weiterhin mindestens eine Kontrollfläche zum Nachweis von Störungen umfaßt. Weiterhin betrifft die Erfindung ein Verfahren zum Nachweis eines oder mehrerer Analyten unter Verwendung einer erfindungsgemäßen Festphase, wobei Störreaktionen erkannt und gegebenenfalls korrigiert werden können.

Beim Nachweis eines Analyten durch Bindungsassays treten bei manchen Proben Störungen in der Nachweisreaktion auf, die zu falschen Messwerten führen. Dieses Phänomen wird meist als Matrixeffekt der Probe bezeichnet. In bisher üblichen Testformaten kann das Vorhandensein einer Störung im allgemeinen nicht angezeigt werden. Durch aufwendige Optimierung von Festphase, Testpuffern und Nachweisreagenz wird versucht, die Matrixeffekte der unterschiedlichen Proben völlig zu unterbinden oder so gering wie möglich zu halten. Eine solche Entstörung von Nachweisverfahren ist jedoch aufwendig und kostspielig. Zudem kann nicht ausgeschlossen werden, dass bei bestimmten Proben trotz Testoptimierung keine ausreichende Entstörung erfolgt, da eine Unterdrückung von Matrixeffekten leider nicht vollständig möglich ist. Weiterhin können neue, bei der Entwicklung des Nachweisverfahrens unbekannte Störungen auftreten, die ebenfalls zu falschen Ergebnissen führen. Folglich besteht das Problem, dass bei den bekannten Nachweisverfahren falsche Testergebnisse erhalten werden können, ohne dass der Anwender dies erkennt. Besonders tragisch ist dieser Umstand bei den qualitativen Tests zum Nachweis einer Infektionskrankheit. So hat zum Beispiel eine aufgrund von Matrixproblemen falsch positive Probe bei einem HIV-Test erhebliche Konsequenzen.

US-A-4,558,013 beschreibt einen Teststreifen, der neben mit spezifischen Testreagenzien beschichteten Testregionen eine nicht begrenzte unbeschichtete, negative Kontrollregion enthält. Der Messwert in der Testregion wird durch Subtraktion der unspezifischen Bindung in der Kontrollregion korrigiert. Durch eine solche Vorgehensweise können Störungen allerdings nur zum Teil korrigiert werden, da sich die unspezifische Bindung von Störkomponenten an die Testregion zumeist beträchtlich von der Bindung von Störkomponenten an die unbeschichtete Kontrollregion unterscheidet.

US-A-5,356,785 beschreibt eine Festphase mit mehreren Testflächen, die jeweils verschiedene Mengen eines Festphasenrezeptors zum Nachweis eines Analyten enthalten. Weiterhin enthält die Festphase eine Referenzfläche, die mit dem Testreagenz ein nachweisbares Signal bekannter Stärke liefert. Kontrollflächen zur Bestimmung unspezifischer Wechselwirkungen zwischen der Probe und der Festphase werden nicht offenbart.

US-A-4,916,056 (Brown III et al.) beschreibt eine Festphase zur qualitativen oder quantitativen Bestimmung eines Analyten, insbesondere eines Antigens, Antikörpers oder eines DNA-Segments in einer Probe. Die Festphase enthält neben einer Testfläche eine Referenzfläche, die ein positives Signal im Test ergibt, sowie eine nicht begrenzte negative Kontrollfläche, in der die positive Referenzfläche und die Testfläche enthalten sind. Ein Nachteil dieser Vorrichtung besteht darin, daß sich die Oberfläche der unbeschichteten Kontrollregion zu stark von der Testfläche unterscheidet, um eine wirksame Korrektur von Störungen zu erreichen.

Eine Aufgabe der Erfindung war es daher, die Vorrichtungen und Verfahren zum Nachweis von Analyten bereitzustellen, die einen direkten Hinweis auf das Vorhandensein und gegebenenfalls die Art von Störungen ermöglichen, so daß diese Störungen bei der Auswertung der Testergebnisse berücksichtigt werden können.

Diese Aufgabe wird erfindungsgemäß gelöst durch ein Verfahren zum Nachweis eines Analyten durch spezifische Bindung des Analyten an einen Festphasenrezeptor, wobei weiterhin Störungen, die durch unspezifische Bindungen von Analyt-fremden Störbestandteilen an die Testfläche hervorgerufen werden, nachgewiesen werden, unter Verwendung einer Festphase mit mindestens einer begrenzten Testfläche, welche einen Festphasenrezeptor mit einer analytspezifischen Rezeptorbindestelle aufweist, wobei die Festphase weiterhin mindestens eine begrenzte Kontrollfläche zum Nachweis von Störungen, die durch unspezifische Bindungen von Analyt-fremden Störbestandteilen an die Testfläche hervorgerufen werden, umfasst, wobei die Kontrollfläche Bestandteile der Testfläche, und zwar Reagenzien der Beladelösung, nicht aber die analytspezifische Rezeptorbindestelle aufweist. Die Erfindung betrifft weiterhin eine Festphase mit mindestens einer begrenztenTestfläche, umfassend einen Festphasenrezeptor, zum Nachweis eines Analyten in einer Probe durch spezifische Bindung des Analyten an eine analytspezifische Rezeptorbindestelle des Festphasenrezeptors, welche dadurch gekennzeichnet ist, dass sie weiterhin mindestens eine begrenzte Kontrollfläche zur Detektion von Störungen umfasst, die durch unspezifische Bindungen von Analyt-fremden Störbestandteilen an die Testfläche hervorgerufen werden, wobei die Kontrollfläche Bestandteile der Testfläche, und zwar Reagenzien der Beladelösung, nicht aber die analytspezifische Rezeptorbindestelle aufweist.

Unter "begrenzten Testflächen" auf einer Festphase ist dabei zu verstehen, daß die Testflächen definierte Bereiche der Festphase umfassen, die vorzugsweise durch inerte Bereiche von weiteren Testflächen räumlich getrennt sind. Die begrenzten Testflächen haben bevorzugt einen Durchmesser von 10 µm bis 1 cm und besonders bevorzugt 10 µm bis 5 mm. Am meisten bevorzugt sind miniaturisierte Testflächen mit einem Durchmesser von 10 µm bis 2 mm. Bevorzugt sind Festphasen mit mehreren Testflächen, die auch als Array-Systeme bezeichnet werden. Solche Array-Systeme sind z.B. bei Ekins und Chu (Clin. Chem. 37 (1995), 1955-1967) und in den US-Patenten 5,432,099, 5,516,635 und 5,126,276 beschrieben. Array-Systeme haben den Vorteil, dass mehrere Analyt- und Kontrollbestimmungen gleichzeitig an einer Probe durchgeführt werden können. Durch die Verwendung von Kontrollflächen zur Detektion von unspezifischen Bindungen und/oder Störproben kann die Ergebnissicherheit gerade bei miniaturisierten Array-Testsystemen erheblich verbessert werden.

Besonders interessant ist dabei die Erfassung von Störungen und unspezifischen Bindungen bei qualitativen Tests und insbesondere bei solchen mit hohen Anforderungen an die Spezifität, wie etwa bei Tests auf Infektionen (z.B. HIV). Durch Anzeigen einer Störung und Korrektur des Meßwerts können falsch positive Ergebnisse deutlich reduziert und somit die Spezifität enorm verbessert werden.

Die erfindungsgemäße Festphase ist ein beliebiger, für Nachweisverfahren gebräuchlicher Träger, vorzugsweise ein nichtporöser Träger, z.B. ein Träger mit Kunststoff-, Glas-, Metall- oder Metalloxidoberfläche. Auch poröse Träger wie etwa Teststreifen sind geeignet. Auf diesem Träger sind räumlich diskrete Bereiche (Testflächen) angeordnet. Auf diesen Testflächen sind immobilisierte Festphasenrezeptoren aufgebracht. Die Immobilisierung der Festphasenrezeptoren erfolgt nach bekannten Methoden, z.B. durch direkte adsorptive Bindung, durch kovalente Kopplung oder durch Kopplung über hochaffine Bindepaare, z.B. Streptavidin/- Biotin, Antigen/Antikörper oder Zucker/Lectin. Durch spezifische Bindung von Komponenten aus dem Nachweismedium, z.B. des zu bestimmenden Analyten oder eines Analytanalogons, an den Festphasenrezeptor kann das Vorhandensein oder/und die Menge des Analyten in einer Probe bestimmt werden.

Der Nachweis des Analyten und des Vorhandenseins von Störreaktionen erfolgt im erfindungsgemäßen Verfahren auf bekannte Weise durch Verwendung von geeigneten Markierungsgruppen, z.B. Fluoreszenzmarkierungsgruppen. Alternativ kann - bei geeigneten Festphasen - die Wechselwirkung von Bestandteilen des Nachweismediums mit den Test- und Kontrollflächen auch durch Bestimmung der Schichtdicke der jeweiligen Fläche, z.B. durch Plasmonenresonanzspektroskopie, nachgewiesen werden.

Bei Array-Systemen, in denen ein gleichzeitiger Nachweis mehrerer Analyten aus einer Probe erfolgt, ist die Verwendung einer "universellen" Markierungsgruppe bevorzugt, mit der ein gleichzeitiger Nachweis mehrerer verschiedener Analyten an verschiedenen Testflächen möglich ist. Ein Beispiel für solche universellen Markierungsgruppen sind Markierungsgruppen, die einen Rezeptor tragen, welcher eine spezifische Wechselwirkung (z.B. über ein hochaffines Bindepaar wie etwa Antikörper/Antigen oder Streptavidin/Biotin etc.) mit einem komplementären Rezeptor auf einem Testreagenz, z.B. einem löslichen Rezeptor für einen zu bestimmenden Analyten oder ein Analytanalogon eingehen kann.

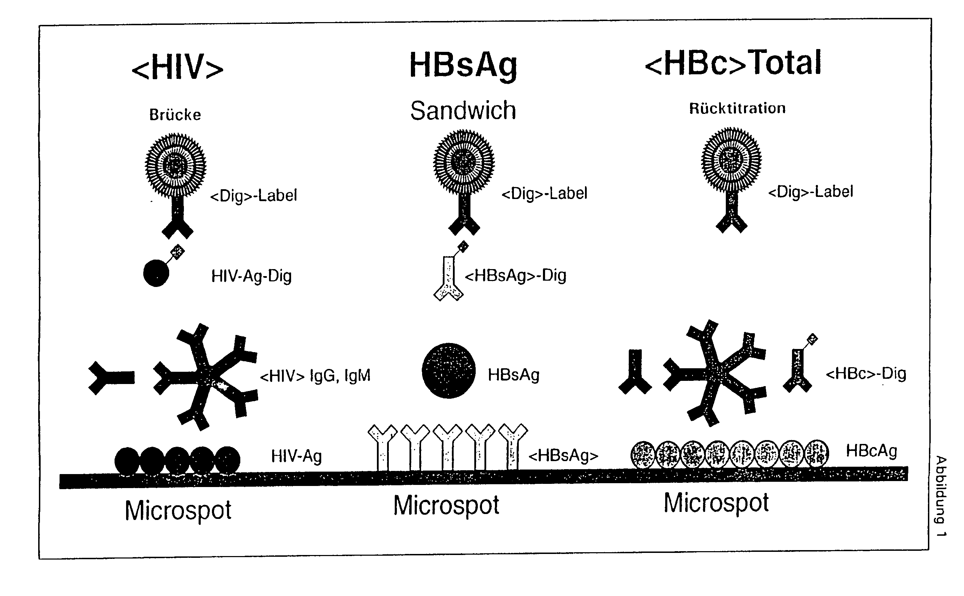

Die Anwendung einer solchen universellen Markierungsgruppe ist exemplarisch in Abbildung 1 erläutert. Dort wird ein fluoreszierendes Latexbead, welches mit einem Anti-Digoxigenin-Antikörper gekoppelt ist (<Dig> Label), für drei verschiedene Testformate auf einer einzigen Festphase, nämlich für die Bestimmung von HIV-Antikörpern, HBs-Antigen und Anti-HBc-Antikörper, eingesetzt. Bei der Anti-HIV-Antikörperbestimmung wird ein immobilisiertes HIV-Antigen und ein digoxigenyliertes lösliches HIV-Antigen verwendet, die mit den nachzuweisenden Anti-HIV-Antikörpern einen immobilisierten Immunkomplex bilden. An die auf diesem Immunkomplex vorhandenen Digoxigeningruppen kann die Markierungsgruppe binden. Bei der Bestimmung von HBs-Antigen werden auf entsprechende Weise ein immobilisierter Antikörper und ein digoxigenylierter löslicher Antikörper, der mit der Markierungsgruppe wechselwirken kann, verwendet. Bei der Anti-HBc-Bestimmung wird ein kompetitives Testformat verwendet, bei dem in der Probe vorhandene Anti-HBc-Antikörper mit einem digoxigenylierten Anti-HBc-Antikörper um immobilisiertes HBc-Antigen konkurrieren. Die an die Testfläche gebundene Menge der Markierungsgruppe ist indirekt proportional zur Anti-HBc-Konzentration in der Probe.

Begrenzte Test- und Kontrollflächen können darüber hinaus zur Unterscheidung gegenüber inerten Bereichen der Festphase eine nachweisbare und analytunspezifische Markierungsgruppe enthalten, die neben der analytspezifischen Markierungsgruppe nachweisbar ist und nicht mit ihr interferiert. Ein Beispiel für eine solche analytunspezifische Markierungsgruppe ist eine Fluoreszenz-Markierungsgruppe, die bei einer Wellenlänge fluoresziert, die von der Fluoreszenzwellenlänge einer analytspezifischen Markierungsgruppe verschieden ist. Die analytunspezifische Markierungsgruppe wird vorzugsweise - ebenso wie der Fesphasenrezeptor - über ein hochaffines Bindepaar, z.B. Streptavidin/Biotin immobilisiert.

Die erfindungsgemäße Festphase kann in beliebigen Nachweisverfahren eingesetzt werden, z.B. in Immunoassays, Nukleinsäure-Hybridisierungsassays, ZuckerLectin-Assays und ähnlichen Verfahren.

Die erfindungsgemäße Festphase ermöglicht selbst dann einen Nachweis von Störreaktionen zum Erhalt von zuverlässigen Testergebnissen, wenn die aus dem Stand der Technik bekannten Entstörungsmaßnahmen für bestimmte Proben nicht ausreichen. Die Kontrollflächen ermöglichen nicht nur einen qualitativen Nachweis von Störreaktionen sondern auch in vielen Fällen eine quantitative Korrektur der Störung.

Zum Nachweis unterschiedlicher Störungen kann die Festphase mehrere, insbesondere unterschiedliche Kontrollflächen zum Nachweis von Störreaktionen umfassen. Auf diese Weise können verschiedene Arten von Störungen spezifisch erfaßt werden. Es empfiehlt sich insbesondere eine Reihe von Kontrollflächen aufzubringen, die zum Nachweis von häufig auftretenden oder/und für den jeweiligen Test besonders relevanten Störkomponenten geeignet sind.

Störungen von Testverfahren können im allgemeinen durch unerwünschte, nicht-analytspezifische Wechselwirkungen von Substanzen auf der Testfläche mit Komponenten des Nachweismediums auftreten. Als Substanzen auf der Testfläche kommen dabei insbesondere Bestandteile der Festphase, Teile des Festphasenrezeptors sowie weitere, sich auf der Oberfläche der Festphase befindende Reagenzien in Betracht. Die störenden Komponenten des Nachweismediums stammen vor allem aus der Probe (Matrixeffekt) und führen in manchen Fällen zur unspezifischen Bindung von Testreagenzien, z.B. dem Nachweisreagenz an die Festphase und führen zur Verfälschung des Meßsignals. Somit kommen störende Wechselwirkungen zwischen der Testfläche und Probekomponenten, Testreagenzien, Reaktionsprodukten oder Komplexen von Probekomponenten und Testreagenzien in Betracht.

Die erfindungsgemäße Festphase umfaßt bevorzugt Kontrollflächen zum Nachweis von Störungen, die durch eine unerwünschte Bindung von Komponenten des Nachweismediums an den für den Analyten spezifischen Festphasenrezeptor hervorgerufen werden. In Proben liegen häufig Analyt-fremde Störbestandteile, wie etwa Antikörper oder Antigene vor, die zu einer erhöhten unspezifischen Bindung mit dem Festphasenrezeptor neigen und auf diese Weise zu fehlerhaften Testergebnissen führen. Besonders vorteilhaft ist deswegen eine Kontrollfläche, welche einen nicht-analytspezifischen Festphasenrezeptor umfaßt, der mit Ausnahme der spezifisch mit dem Analyten bindefähigen Region völlig identisch mit dem Festphasenrezeptor in den Testflächen ist.

Wenn der Festphasenrezeptor beispielsweise ein Antikörper oder ein Antikörperfragment ist, verwendet man eine Kontrollfläche, die einen unspezifischen Antikörper oder ein unspezifisches Antikörperfragment der gleichen Spezies, bevorzugt der gleichen Klasse und besonders bevorzugt der gleichen Subklasse wie der Festphasenrezeptor der Testfläche enthält. Wenn der Festphasenrezeptor beispielsweise ein Antigen, z.B. ein Peptid oder ein Polypeptid ist, verwendet man eine Kontrollfläche, die ein mutiertes "Antigen" enthält, welches sich von einem immunologisch reaktiven Antigen durch Veränderungen, z.B. durch Veränderung einer möglichst geringen Anzahl Aminosäuren im Bereich immunogener Epitope unterscheidet. Diese Aminosäureveränderungen können Insertionen, Deletionen und vorzugsweise Substitutionen natürlicher Aminosäuren durch andere natürliche Aminosäuren oder nicht natürliche Aminosäurederivate, z.B. D-Aminosäuren umfassen. Wenn der Festphasenrezeptor beispielsweise eine Nukleinsäure ist, verwendet mann eine Kontrollfläche, die eine "mutierte" Nukleinsäure enthält, die sich von der auf der Testfläche immobilisierten Nukleinsäure durch Veränderungen in der Nukleotidsequenz, beispielsweise durch einen Basenaustausch innerhalb der für die Erkennung einer Zielnukleinsäure verantwortlichen Sequenz, vorzugsweise in deren Mitte, unterscheidet. Am meisten bevorzugt wird ein Mutein des Festphasenrezeptors verwendet, welches mit Ausnahme der analytspezifischen Antigenbindungsstelle völlig identisch mit dem Festphasenantikörper in den Testflächen ist.

Bevorzugt ist auch, wenn der auf die Kontrollfläche aufgebrachte Festphasenrezeptor identische Behandlungsschritte, z.B. Derivatisierungen, wie der auf die Testfläche aufgebrachte Festphasenrezeptor durchlaufen hat. So sollte, beispielsweise - bei einem biotinylierten Festphasenrezeptor - die Zahl der an den Festphasenrezeptor angekoppelten Biotinmoleküle in der Testfläche und der Kontrollfläche gleich sein. Weiterhin sollten die Festphasenrezeptoren in der Testfläche und der Kontrollfläche eine identische Kopplungschemie durchlaufen haben. Außerdem sollten auch identische Linker verwendet werden. Ein für eine Kontrollfläche geeigneter Festphasenrezeptor darf keine spezifische Bindefähigkeit für den Analyten aufweisen, umfasst aber vorzugsweise alle anderen Bereiche und somit Bindestellen des Festphasenrezeptors der Testfläche. Somit ist die unspezifische Bindung von Störkomponenten an den jeweiligen Festphasenrezeptor der Testfläche und Kontrollfläche im wesentlichen identisch, so dass eine quantitative Korrektur des Meßwertes der Testfläche anhand des Meßwertes der Kontrollfläche vorgenommen werden kann.

Bevorzugt umfaßt die Festphase weiterhin mindestens eine Kontrollfläche zum Nachweis von Störungen, die durch die Reaktion anderer immobilisierter Reagenzien in den Testflächen mit Nicht-Analytkomponenten der Probe hervorgerufen werden. Dadurch werden insbesondere Störkomponenten erfaßt, die spezifisch oder/und unspezifisch mit Komponenten der zum Aufbringen des Festphasenrezeptors verwendeten Beladelösung reagieren. Eine solche Kontrollfläche kann beispielsweise bei einem Testsystem verwendete Reagenzien der Beladelösung wie etwa Puffer, Immobilisierungsreagenzien, wie Streptavidin, biotinylierte Substanzen, z.B. biotinylierte Fluoreszenzmarker, nicht-analytspezifische Antikörper etc., Blockierungsreagenzien oder Linker, enthalten.

Oftmals werden Tests durch Rheumafaktoren gestört. Daher ist es bevorzugt, mindestens eine Kontrollfläche zum Nachweis auf Rheumafaktoren auf die Festphase aufzubringen. Rheumafaktoren sind zumeist IgM-Moleküle, seltener auch IgG-, IgA- und IgE-Moleküle, die mit dem Fc-Teil von Antikörpern reagieren und - wenn sie z.B. eine Kreuzreaktion mit dem auf der Testfläche immobilisierten Antikörper oder/und einem löslichen Nachweisantikörper zeigen - den Test stören, z.B. durch Vernetzung des Festphasen-gebundenen Antikörpers mit einem markierten Nachweisantikörper, wodurch ein unspezifisch erhöhtes Signal erhalten wird. Für eine entsprechende Kontrollfläche wird ein unspezifisches IgG-Molekül, bevorzugt der Fcγ-Teil eines humanen IgG-Moleküls auf die Festphase aufgebracht. Während des Tests bindet dann der Rheumafaktor nicht nur an die Testfläche, sondern auch an die Kontrollfläche und zeigt so die Störung an.

Eine weitere relativ häufig auftretende Störung wird durch fremdspezies-spezifische Antikörper in der Probe, d.h. Antikörper, die gegen Antikörper fremder Spezies gerichtet sind, z.B. humane Anti-Maus-Antikörper (HAMA), hervorgerufen. Daher umfaßt die erfindungsgemäße Festphase bevorzugt auch mindestens eine Kontrollfläche zum Test auf fremdspezies-spezifische Antikörper. Fremdspezies-spezifische Störkomponenten, z.B. HAMA führen beispielsweise in einem Doppel-Antikörper-Sandwich-Assay zu einer Vernetzung des Festphasen-Antikörpers mit dem Nachweis-Antikörpe- und folglich zu einem unspezifisch erhöhten Signal. Für eine geeignete Kontrollfläche wird bevorzugt ein unspezifischer Antikörper derselben Spezies wie der Testantikörper verwendet. Eine möglicherweise in der Probe vorhandene HAMA-Störkomponente bindet an den auf die Kontrollfläche aufgebrachten Antikörper, wodurch die Störreaktion angezeigt wird.

Anstelle eines Festphasenrezeptors, welcher mit dem Nachweisreagenz in der Testfläche, abgesehen von der spezifisch analytbindefähigen Region, identisch ist, kann auf eine Kontrollfläche auch der bei der Nachweisreaktion gebildete Komplex, z.B. Festphasenantikörper-Analyt-Nachweisantikörper (ohne Markierung), aufgebracht werden, so dass keine spezifische Reaktion mehr möglich ist, während die unspezifischen Bindungsstellen nahezu identisch mit denen der Testfläche sind.

Es sind auch Störkomponenten in Serum bekannt, die gegen Neo-Epitope eines Antikörperfragments gerichtet sind. Solche Neo-Epitope entstehen z.B. bei der F(ab')₂-Spaltung eines IgG-Moleküls. Hier ist es bevorzugt, eine Kontrollfläche vorzusehen, die ein Fragment eines unspezifischen Antikörpers enthält, welches mit derselben Methode (Spaltbedingungen und Spaltprotein) hergestellt wurde, wie die Antikörperfragmente der Testfläche. Die Störkomponenten binden an diese unspezifischen Antikörperfragmente, wodurch sie nachgewiesen werden können.

Außerdem besteht die Möglichkeit, mit geeigneten Kontrollflächen auch gegebenenfalls in einer Probe vorliegende Störkomponenten, z. B. Störantikörper zu erfassen, die gegen Immobilisierungsreagenzien auf den Testflächen wie Streptavidin gerichtet sind. Hierzu wird Streptavidin (SA) auf eine Kontrollfläche aufgebracht und dem Nachweisreagenz markiertes SA zugesetzt. Bei Anwesenheit von Anti-SA-Antikörpern in der Probe wird ein Sandwichkomplex ausgebildet und der Antikörper spezifisch nachgewiesen.

Weiterhin kann die erfindungsgemäße Festphase eine Kontrollfläche zum Nachweis des Gesamt-IgE-Gehalts enthalten. Eine solche Kontrollfläche ist insbesondere bei Allergie-Tests empfehlenswert. Bei der spezifischen IgE-Bestimmung in der Allergiediagnostik wird die spezifische Nachweisreaktion eines bestimmten IgE oftmals durch Proben mit hohem Gesamt-IgE-Gehalt gestört. Mit Hilfe einer Kontrollfläche, auf die Anti-IgE-Antikörper aufgebracht worden sind, kann parallel zum Nachweisverfahren der Gesamt-IgE-Gehalt bestimmt werden.

Schließlich können auch Kontrollflächen zum Nachweis von Störungen vorgesehen werden, die durch Reaktionen von Komponenten des Nachweismediums mit dem Festphasenträger hervorgerufen werden. Hierzu werden alle Bestandteile des Festphasenträgers, wie Trägermaterial (z.B. Polystyrol), Weichmacher, funktionelle Gruppen usw. auf eine Kontrollfläche aufgebracht.

Außerdem können bei bestimmten Testverfahren, z.B. bei qualitativen oder semiquantitativen Tests auf Infektionskrankheiten, Allergien etc. Kontrollflächen zur Ermittlung eines Cut-off Werts verwendet werden. Der "Cut-off"-Wert ist ein Grenzwert, der bei Testverfahren gesetzt wird, um zwischen positiven und negativen Werten unterscheiden zu können. Ein solcher "Cut-off'-Wert ist insbesondere bei Testverfahren, welche Infektionskrankheiten betreffen, von Bedeutung. Zum einen kann eine "Negativ"-Kontrollfläche verwendet werden, die eine Beladelösung ohne das Testreagenz oder ein Mutein des Testreagenzes enthalten kann. Der große Vorteil liegt darin, daß für jede Probe ein probenspezifischer Wert der unspezifischen Bindung erfaßt wird und somit eine verbesserte Spezifität des Tests erhalten wird. Auf diese Weise kann eine separate Negativkontrolle entfallen.

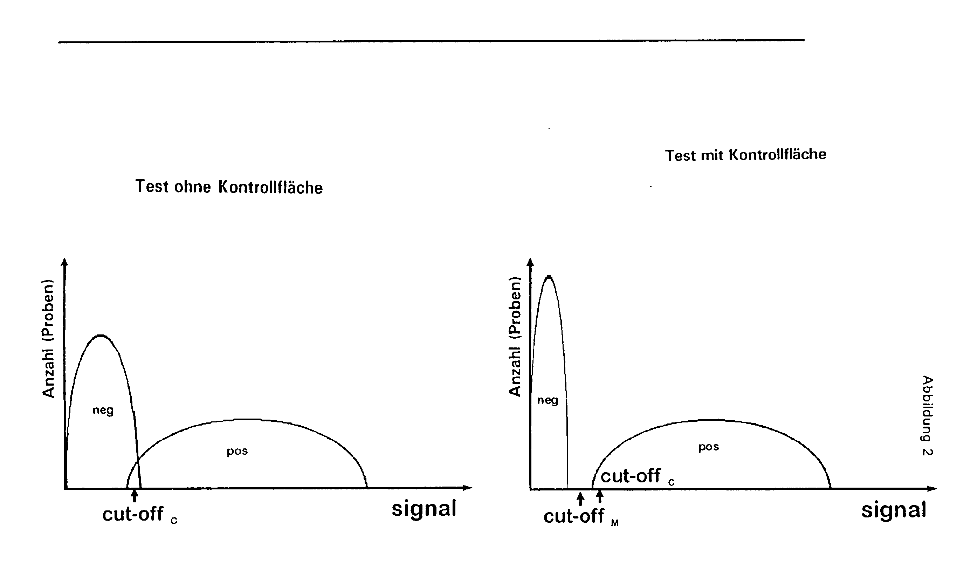

Abbildung 2 zeigt die Wirkung einer Kontrollfläche beim Einsatz in einem Test, bei dem ein Cut-off-Wert verwendet wird. Bei einer großen Anzahl von Proben wird bei einer konventionellen Testdurchführung eine gewisse Anzahl falsch negativer und falsch positiver Ergebnisse erhalten (linke Seite). Bei der erfindungsgemäßen Verwendung von Kontrollflächen (rechte Seite) kann eine selektive Verringerung der Signale aus negativen Proben erzielt werden, was zu einer Verringerung des Cut-off-Werts führt. Auf diese Weise kann eine positiv/negativ Unterscheidung mit erheblich geringerer Fehlerwahrscheinlichkeit getroffen werden.

Auch eine Positivkontolle kann durch eine Referenzfläche, die den Analyten oder ein analytähnliches Reagenz enthält, simuliert werden. Der große Vorteil liegt darin, daß mit der Kombination von einer Negativ-Kontrollfläche mit einer Positiv-Referenzfläche ein probenspezifischer Cut-off-Wert für jede einzelne Messung bestimmt werden kann. Dies hat den Vorteil, daß keine indirekte Kalibrierung notwendig ist und eine bessere Testspezifität erreicht wird.

Es ist selbstverständlich möglich, neben den oben genannten bevorzugten Kontrollflächen je nach Testführung andere oder/und weitere Kontrollflächen vorzusehen, um in einer Probe vorhandene oder vermutete Störkomponenten zu erfassen.

Die erfindungsgemäß vorgesehenen Kontrollflächen ermöglichen nicht nur eine qualitative Erkennung von Störreaktionen, sondern oftmals auch eine quantitative Korrektur der Störung. Bei geeigneter Wahl der Testbedingungen wird gerade die in den Testflächen auftretende unspezifische Bindung von Nicht-Analytkomponenten in bestimmten Kontrollflächen abgebildet. Somit kann der Messwert in einer Testfläche einfach korrigiert werden, wodurch ein korrektes und unverfälschtes Ergebnis erhalten wird. Auch bei nicht identischer unspezifischer Bindung in der Testfläche und der Kontrollfläche, kann eine Korrektur des Meßsignals bei qualitativen Tests erfolgen, da hier nur eine Aussage über "positiv" oder "negativ" notwendig ist.

Ein weiterer Vorteil besteht darin, dass es mit der erfindungsgemäßen Festphase möglich ist, ohne großen Aufwand das Vorliegen mehrerer Störkomponenten separat zu erkennen und es möglich ist, die Art der Störung zu bestimmen. Der Anwender erkennt unmittelbar, dass das Ergebnis aufgrund des Vorliegens einer oder mehrerer Störkomponenten verfälscht sein könnte. Der Anwender kann dann entweder anhand der ermittelten Daten die Messergebnisse korrigieren, die Messung mit einem anderen Testformat wiederholen oder die Probe in geeigneter Weise vorbehandeln, z.B. durch Abtrennung der Störkomponenten.

Die erfindungsgemäße Festphase kann zum Nachweis eines Analyten in einer Probe, z.B. in einer Körperflüssigkeit, wie etwa Blut, Serum, Plasma, Speichel etc. verwendet werden. Vorzugsweise werden Festphasen mit mehreren Testflächen, d.h. Array-Systeme, zum gleichzeitigen Nachweis mehrerer Analyten verwendet. Vorzugsweise wird in solchen Array-Systemen für jede Testfläche mindestens eine Kontrollfläche verwendet. Die erfindungsgemäßen Festphasen können in allen bekannten heterogenen Testverfahren eingesetzt werden, insbesondere bei Immunoassays und Nukleinsäurehybridisierungsassays.

Ein weiterer Gegenstand der Erfindung ist somit ein Verfahren zum Nachweis eines Analyten durch spezifische Bindung des Analyten an einen Festphasenrezeptor, wobei weiterhin Störungen, die durch unspezifische Bindungen von Analyt-fremden Störbestandteilen an die Testfläche hervorgerufen werden, nachgewiesen werden, unter Verwendung einer Festphase mit mindestens einer begrenzten Testfläche, welche einen Festphasenrezeptor mit einer analytspezifischen Rezeptorbindestelle aufweist, wobei die Festphase weiterhin mindestens eine begrenzte Kontrollfläche zum Nachweis von Störungen, die durch unspezifische Bindungen von Analyt-fremden Störbestandteilen an die Testfläche hervorgerufen werden, umfasst, wobei die Kontrollfläche Bestandteile der Testfläche, und zwar Reagenzien der Beladelösung, nicht aber die analytspezifische Rezeptorbindestelle aufweist. Vorzugsweise enthält das erfindungsgemäße Verfahren die Verwendung der Kontrollflächen zur quantitativen Korrektur von Störungen.

Schließlich betrifft die Erfindung auch die Verwendung von Kontrollflächen in einem Verfahren zum Nachweis eines Analyten zur gleichzeitigen Erkennung von Störungen, nämlich die Verwendung von begrenzten Kontrollflächen zur Erkennung von Störungen, die durch Analyt-fremde Bestandteile hervorgerufen werden, in einem Verfahren zum Nachweis eines Analyten durch spezifische Bindung des Analyten an eine analytspezifische Rezeptorbindestelle eines Festphasenrezeptors in einer begrenzten Testfläche, wobei die Kontrollfläche Bestandteile der Testfläche, und zwar Reagenzien der Beladelösung, nicht aber die analytspezifische Rezeptorbindestelle aufweist.

Die Erfindung wird durch die nachfolgenden Abbildungen und Beispiele näher erläutert. Es zeigen:
- Abbildung 1:: ein Beispiel für ein miniaturisiertes Array-System (Microspot), das unter Verwendung einer universellen Markierungsgruppe die gleich- zeitige Bestimmung von 3 Parametern auf einer Festphase erlaubt;
- Abbildung 2:: die Spezifitätsverbesserung, die in Testformaten, bei denen ein Cut- off-Wert bestimmt wird, durch die erfindungsgemäße Verwendung von Kontrollflächen erzielt wird und
- Abbildung 3:: die schematische Darstellung einer erfindungsgemäßen Festphase zur Bestimmung von HBs-Antigen, die neben der Testfläche (<HBsAg>) zusätzlich mehrere Kontrollflächen (<CK-MB>; <TNT> und <TSH>) enthält.

### Beispiele

### Beispiel 1 Durchführung eines HBsAg-Tests

Auf einen Polystyrolträger wird auf eine Testfläche von ca. 100 *µ*m ein monoklonaler Antikörper gegen HBsAg aufgebracht. Durch mehrmaliges Aufbringen der identischen Reagenzlösung kann ohne Mehraufwand bei der Testdurchführung pro Probenpipettierung eine Mehrfachbestimmung durchgeführt werden. Auf die Testfläche werden 30 *µ*l mit Probenpuffer vorverdünnte Probe pipettiert und 20 Minuten unter Schütteln bei Raumtemperatur inkubiert. Nach Absaugen der Probe und Waschen des Testfeldes mit Waschpuffer werden 30 *µ*l Reagenzlösung 1 mit Digoxigenin (Dig)-markiertem Anti-HBsAg-Antikörper zupipettiert und wiederum 20 Minuten unter Schütteln bei Raumtemperatur inkubiert. Nach Absaugen der Reagenzlösung 1 und Waschen des Testfeldes mit Waschpuffer werden 30 *µ*l Reagenzlösung 2 mit Nachweisreagenz auf das Testfeld pipettiert. Als Nachweisreagenz dienen 100 nm große, fluoreszenzgefärbte Latexpartikel, die kovalent mit einem Anti-Dig-Antikörper beschichtet sind. Dieses Nachweisreagenz wird wiederum 20 Minuten unter Schütteln bei Raumtemperatur inkubiert, anschließend abgesaugt, gewaschen und trockengesaugt. Das Testfeld wird mit einem He-Ne-Laser mit 633 nm Wellenlänge bestrahlt und die Fluoreszenz bei 670 nm Wellenlänge mit einer CCD-Kamera vermessen. Eine schematische Darstellung dieses Testformates ist in Abb. 1 Mitte gezeigt.

Folgende testspezifischen Reagenzien wurden verwendet:
- Festphasenantikörper:: Monoklonaler Maus Anti-HBsAg-Antikörper-1 (Fab'₂- Fragment)-Biotin-Konjugat 1:1 Subtyp IgG1
- Nachweisantikörper:: Monoklonaler Maus Anti-HBsAg-Antikörper-2 IgG-Dig- Konjugat 1:10

Folgende Meßwerte (Counts) wurden gemessen:

| Probe | Negativ-Kontrollfeld (Counts) | Signal Testfeld (Counts) | Signal Testfeld - Negativ-Kontrollfeld | Cut-off-Index** |
|---|---|---|---|---|
| Negativkontrolle | 22 | 22 | 0 | 0,0 |
| Positivkontr.1 (20 U/ml) | 20 | 5459 | 5439 | 82,4 |
| Positivkontr.2 (3 U/ml) | 27 | 760 | 733 | 11,1 |
| Negativprobe 1 | 15 | 15 | 0 | 0,0 |
| Negativprobe 2 | 15 | 15 | 0 | 0,0 |
| Negativprobe 3 | 14 | 14 | 0 | 0,0 |
| Negativprobe 4 | 15 | 15 | 0 | 0,0 |
| Negativprobe 5 | 15 | 15 | 0 | 0,0 |
| Negativprobe 6 | 17 | 17 | 0 | 0,0 |

| | | | | |
|---|---|---|---|---|
| * Negativ-Kontrollfeld entspricht unspezifischer Bindung im Kontrollfeld ohne Festphasenrezeptor ** Cut-off-Index: [Signal (Testfeld)-Signal (Negativ-Kontrollfeld)]/3 x Signal(Negativ-Kontrolle) | | | | |

Bei einem Cut-off > 1 ist der Test positiv. Bei einem Cut-off < 1 ist der Test negativ.

### Beispiel 2: HBsAg-Test mit Kontrollspots zur Detektion von HAMA-Störern

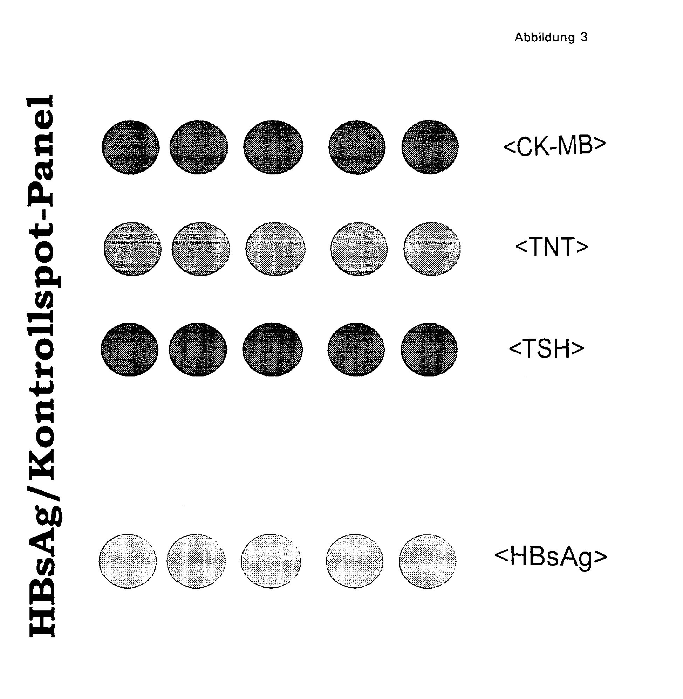

Zur Detektion von HAMA-Störern wurden neben HBsAg-spezifischem Antikörper weitere unspezifische monoklonale Antikörper (MAKs) zur Detektion von HAMA-Störern aufgebracht (siehe Abbildung 3). Dabei wurden Antikörper gegen Creatinkinase-MB (<CK-MB>), Troponin T (<TNT>) und Thyroid-stimulierendes Hormon (<TSH>) in Form von Fab'₂- oder Fab'-Biotin-Konjugaten eingesetzt. Aufgrund der Tatsache, daß die humanen Anti-Maus-Antikörper in der Probe eine Vernetzung des an der Festphase gebundenen unspezifischen MAKs und des spezifischen Nachweisantikörpers hervorrufen, kann die sogenannte HAMA-Störung nachgewiesen werden. Ziel des Versuches war es, den optimalen MAK zur HAMA-Detektion zu finden. Das HBsAg/Kontroll-Panel wurde mit 6 verschiedenen HAMA-Proben vermessen. Dazu wurden dem Probenpuffer kein bzw. 100 µg/ml HAMA-Entstörreagenz zugesetzt.

### a) Versuchsergebnisse ohne HAMA-Entstörreagenz

| Probe | MAK<HBsAg> F(ab')₂-Bi 1:1* (Counts) | MAK<TSH> F(ab')₂-Bi 1:1* (Counts) | MAK<TNT> Fab'-Bi 1:1* (Counts) | MAK<CK-MB> Fab'-Bi1:1* (Counts) | Cut-off- Index** HBsAg |
|---|---|---|---|---|---|
| Neg-Kontr | 3 | 18 | 0 | 10 | 0,04 |
| Pos-Kontr (20 U/ml) | 6169 | 23 | 0 | 5 | 73,4 |
| HAMA 1 | 1598 | 2808 | 741 | 177 | 19,0 |
| HAMA 2 | 4889 | 10434 | 306 | 42 | 58,2 |
| HAMA 3 | 737 | 3728 | 29 | 32 | 8,8 |
| HAMA 4 | 358 | 2513 | 0 | 0 | 4,3 |
| HAMA 5 | 43 | 2565 | 0 | 0 | 0,5 |
| HAMA 6 | 32157 | 32108 | 32717 | 32930 | 382,8 |

| | | | | | |
|---|---|---|---|---|---|
| * Signal (Testfeld)-Signal (Neg.-Kontrollfeld) ** Cut-off-Index = [Signal (Testfeld)-Signal (Negativ-Kontrollfeld)]/3 x Signal (Negativkontrolle) Signal (Negativkontrolle) = 28 Counts | | | | | |

Dieses Ergebnis zeigt, daß der HBsAg-Test durch 5 der 6 HAMA-Proben stark gestört wird und zu einem falsch positiven Ergebnis führt. Durch die Anwesenheit der Kontrollspots kann die Störung eindeutig angezeigt werden. Am besten eignet sich dafür der TSH-MAK, der das identische Spaltfragment (Fab'₂), die identische Biotin-Stöchiometrie und die identische Kopplungschemie aufweist. Die beiden MAKs mit Fab'-Fragmenten reagieren dagegen schlechter als der HBsAg-MAK mit den HAMA-Störproben und sind deshalb zur Identifizierung von allen HAMA-Störern weniger geeignet.

### b) Versuchsergebnisse mit 100 µg/ml HAMA-Entstörreagenz

| Probe | MAK<HBsAg> F(ab')₂-Bi 1:1* (Counts) | MAK<TSH> F(ab')₂-Bi 1:1* (Counts) | MAK<TNT> Fab'-Bi 1:1* (Counts) | Cut-off- Index** HBsAg | Cut-off- Index HBsAg_{korr.}^{***} |
|---|---|---|---|---|---|
| Neg-Kontr | 0 | 0 | 0 | 0,0 | 0,0 |
| Pos-Kontr (20 U/ml) | 4186 | 0 | 0 | 41,0 | 41,0 |
| HAMA 1 | 0 | 0 | 0 | 0,0 | 0,0 |
| HAMA 2 | 0 | 0 | 0 | 0,0 | 0,0 |
| HAMA 3 | 0 | 0 | 0 | 0,0 | 0,0 |
| HAMA 4 | 0 | 0 | 0 | 0,0 | 0,0 |
| HAMA 5 | 0 | 0 | 0 | 0,0 | 0,0 |
| HAMA 6 | 144 | 187 | 217 | 1,4 | 0,0 |

| | | | | | |
|---|---|---|---|---|---|
| * Signal (Testfeld) - Signal (Negativkontrollfeld) **Cut-off-Index = [Signal (Testfeld) - Signal (Negativ-Kontrollfeld)]/3 x Signal (Negativkontrolle) Signal (Negativkontrolle) = 34 Counts *** Cut-off-Index HBsAgₖₒᵣᵣ = [Signal (Testfeld) - Signal (MAK-Kontrollfeld]/3x Signal (Negativkontrolle); Definition: negative Werte = 0 | | | | | |

Durch den Zusatz von einer sehr großen Menge von 100 *µ*g/ml Entstörreagenz, die den Test für Routineanwendungen zu teuer machen würde, können zwar 5 der 6 HAMA-Proben entstört werden. Ein Problem bleibt alldings die HAMA-Probe 6, die trotz der hohen Konzentration an Entstörreagenz nicht entstört werden kann. Mit Hilfe der beiden Kontrollspots kann die noch vorhandene HAMA-Störung deutlich angezeigt werden. Da die unspezifische Bindung im TSH-Kontrollspot der unspezifischen Bindung im spezifischen HBsAg-Spot entspricht, kann das spezifische Signal mit Hilfe des Signals im Kontrollspot korrigiert werden. Mit dieser Maßnahme wird auch diese Probe eindeutig negativ, was zu einer deutlichen Verbesserung der Spezifität führt. Daher können mit Hilfe der Kontrollspots unter Verbesserung der Spezifität die Konzentrationen an Entstörprotein im Probenpuffer und somit die Herstellungskosten deutlich gesenkt werden.

### Beispiel 3: HBsAg-Test mit Kontrollspots zur Detektion von probenspezifischen Matrixeffekten

Es ist allgemein bekannt, daß in Bindungsassays durch unterschiedliche Matrixeffekte unspezifische Bindungen des Nachweisreagenz hervorgerufen werden können. Durch das Aufbringen von Kontrollspots, die ein vergleichbares Verhalten gegenüber Matrixeffekten wie der testspezifische Spot aufweisen, ist es möglich, solche Matrixeffekte anzuzeigen und gegebenenfalls sogar zu korrigieren. Dies führt folglich zu einer deutlichen Verbesserung der Spezifität.

Im nachfolgenden Versuch wurde wiederum ein HBsAg-Test mit verschiedenen HBsAg-negativen Proben vermessen, die aufgrund von auffälligen Matrixeffekten ausgewählt wurden.

| Probe | MAK<HBsAg> F(ab')₂-Bi 1:1* (Counts)* | MAK<TSH> F(ab')₂ Bi 1:1* (Counts) | Cut-off-Index** HBsAg | Cut-off-Index HBsAgₖₒᵣᵣ^{***} |
|---|---|---|---|---|
| Neg-Kontrolle | 3 | 18 | 0,04 | 0,0 |
| Pos-Kontrolle (20 U/ml) | 6169 | 23 | 73,4 | 73,2 |
| Negativprobe 3415 | 0 | 0 | 0,0 | 0,0 |
| Negativprobe 3418 | 65 | 102 | 0,77 | 0,0 |
| Negativprobe 4561 | 128 | 494 | 1,52 | 0,0 |
| Negativprobe 4567 | 158 | 328 | 1,88 | 0,0 |
| Negativprobe 4609 | 0 | 0 | 0,0 | 0,0 |
| Negativprobe S21 | 25 | 52 | 0,30 | 0,0 |
| Negativprobe S25 | 4 | 3 | 0,05 | 0,01 |
| Negativprobe S45 | 16 | 30 | 0,19 | 0,0 |
| Negativprobe S49 | 0 | 0 | 0,0 | 0,0 |

| | | | | |
|---|---|---|---|---|
| * Signal (Testfeld) - Signal (Negativkontrollfeld) ** Cut-off-Index = [Signal (Testfeld) - Signal (Negativkontrollfeld)]/3 x Signal (Negativkontrolle) Signal (Negativkontrolle) = 28 Counts *** Cut-off-Index HBsAgₖₒᵣᵣ. = [Signal (Testfeld) - Signal (MAK-Kontollfeld]/3 x Signal (Negativkontrolle); Definition negative Werte = 0 | | | | |

Von den vermessenen 9 auffälligen Negativproben führen 2 Proben zu falschpositiven Ergebnissen. Durch Korrektur des Meßwerts mit Hilfe des Kontrollspots werden alle Proben auf Null gebracht und sind somit eindeutig negativ.

### Beispiel 4: HBsAg-Test mit Kontrollflächen zur Detektion von Störungen durch Rheumafaktoren

Zur Detektion einer Störung durch Rheumafaktoren wurden neben dem HBsAgspezifischen Antikörper weitere MAKs vom Subtyp IgG1 aufgebracht. Aufgrund der Tatsache, daß Rheumafaktoren mit dem Fc-Teil von Antikörpern reagieren, kann eine Vernetzung des an der Festphase gebundenen testspezifischen MAKs und des spezifischen Nachweisantikörpers hervorgerufen und somit eine falsch positive Reaktion erzeugt werden. Ziel des Versuches war es, den optimalen MAK zur Detektion von Rheumafaktoren zu finden. Aus diesem Grund wurden unterschiedliche MAKs der Subklasse IgG1 aufgetragen Dieses HBsAg/Kontroll-Panel wurde mit einer Negativkontrolle, 3 verschiedenen Positivkontrollen, 4 normalen Negativproben und 5 Negativproben mit ansteigenden Rheumafaktoren (RF 1-5) vermessen. Der jeweilige Gehalt der Positivkontrollen an HBsAg und der Gehalt der Rheumafaktorproben an Rheumafaktoren ist in U/ml angegeben. Die Ergebnisse waren wie folgt:

| Probe | MAK <HBsAg> IgG-Bi [Counts] | MAK<lgE> "1"-IgG-Bi [Counts] | MAK<IgE> "2"-IgG-Bi [Counts] | MAK<IgE> "3"-IgG-Bi [Counts] | Negativ-kontrollfeld* [Counts] | Cut-off- Index* HBsAg |
|---|---|---|---|---|---|---|
| Neg-Kontr | 27 | 34 | 31 | 30 | 27 | 0,0 |
| Pos-Kontr (20 U/ml) | 4701 | 44 | 44 | 44 | 44 | 57,5 |
| Pos-Kontr (5 U/ml) | 842 | 43 | 43 | 43 | 43 | 9,9 |
| Pos-Kontr (0,1 U/ml) | 106 | 31 | 31 | 31 | 31 | 0,9 |
| Neg-Probe1 | 30 | 35 | 30 | 30 | 30 | 0,0 |
| Neg-Probe2 | 28 | 33 | 33 | 34 | 28 | 0,0 |
| Neg-Probe3 | 29 | 35 | 29 | 29 | 29 | 0,0 |
| Neg-Probe4 | 27 | 32 | 27 | 27 | 27 | 0,0 |
| RF 1 (307 U/ml) | 39 | 34 | 29 | 30 | 27 | 0,15 |
| RF 2 (421 U/ml) | 41 | 33 | 28 | 28 | 28 | 0,16 |
| RF 3 (1307 U/ml) | 429 | 33 | 115 | 64 | 30 | 4,9 |
| RF 4 (1793 U/ml) | 5790 | 47 | 865 | 204 | 47 | 70,9 |
| RF 5 (2599 U/ml) | 7530 | 106 | 2527 | 759 | 54 | 92,3 |

| | | | | | | |
|---|---|---|---|---|---|---|
| * Cut-off-Index = [Signal (Testfeld)-Signal (Negativ-Koontrollfeld)]/3 x Signal (Negativkontrolle) Cut-off-Index > 1 = positiv | | | | | | |

Dieses Beispiel zeigt deutlich, daß Negativproben mit einer Konzentration an Rheumafaktoren > 1000 U/ml den HBsAg-Test deutlich stören und ein falsch positives Ergebnis liefern. 2 der 3 geprüften MAK-Kontrollflächen simulieren die Störung des HBsAg-Tests und liefern ebenfalls deutlich positive Reaktionen mit den 3 hochtitrigen Rheumaseren. Auf diese Weise wird die Teststörung sofort erkannt, so daß ein falschpositives Ergebnis vermieden wird. Am besten ist der MAK "2" geeignet, um die Rheumafaktorstörung anzuzeigen.

## Patentansprüche

1. Verfahren zum Nachweis eines Analyten durch spezifische Bindung des Analyten an einen Festphasenrezeptor, wobei weiterhin Störungen, die durch unspezifische Bindungen von Analyt-fremden Störbestandteilen an die Testfläche hervorgerufen werden, nachgewiesen werden, unter Verwendung einer Festphase mit mindestens einer begrenzten Testfläche, welche einen Festphasenrezeptor mit einer analytspezifischen Rezeptorbindestelle aufweist, wobei die Festphase weiterhin mindestens eine begrenzte Kontrollfläche zum Nachweis von Störungen, die durch unspezifische Bindungen von Analyt-fremden Störbestandteilen an die Testfläche hervorgerufen werden, umfasst, wobei der Festphasenrezeptor mit der analytspezifischen Rezeptorbindestelle unter Verwendung einer Beladelösung aufgebracht wurde und wobei die Kontrollfläche Bestandteile der Testfläche, und zwar Reagenzien der Beladelösung, nicht aber die analytspezifische Rezeptorbindestelle aufweist.

2. Verfahren nach Anspruch 1,
**dadurch gekennzeichnet,**
**dass** die Kontrollflächen zur quantitativen Korrektur von Störungen verwendet werden.

3. Verwendung von begrenzten Kontrollflächen zur Erkennung von Störungen, die durch Analyt-fremde Bestandteile hervorgerufen werden, in einem Verfahren zum Nachweis eines Analyten durch spezifische Bindung des Analyten an eine analytspezifische Rezeptorbindestelle eines Festphasenrezeptors in einer begrenzten Testfläche, wobei der Festphasenrezeptor mit der analytspezifischen Rezeptorbindestelle unter Verwendung einer Beladelösung aufgebracht wurde und wobei die Kontrollfläche Bestandteile der Testfläche, und zwar Reagenzien der Beladelösung, nicht aber die analytspezifische Rezeptorbindestelle aufweist.

4. Festphase mit mindestens einer begrenzten Testfläche, umfassend einen Festphasenrezeptor, zum Nachweis eines Analyten in einer Probe durch spezifische Bindung des Analyten an eine analytspezifische Rezeptorbindestelle des Festphasenrezeptors,
**dadurch gekennzeichnet,**
**dass** sie weiterhin mindestens eine begrenzte Kontrollfläche zur Detektion von Störungen umfasst, die durch unspezifische Bindungen von Analyt-fremden Störbestandteilen an die Testfläche hervorgerufen werden, wobei der Festphasenrezeptor mit der analytspezifischen Rezeptorbindestelle unter Verwendung einer Beladelösung aufgebracht wurde und wobei die Kontrollfläche Bestandteile der Testfläche, und zwar Reagenzien der Beladelösung, nicht aber die analytspezifische Rezeptorbindestelle aufweist.

5. Festphase nach Anspruch 4,
**dadurch gekennzeichnet,**
**dass** sie mehrere Testflächen zum gleichzeitigen Nachweis mehrerer Analyten in einer Probe umfaßt.

6. Festphase nach einem der Ansprüche 4 oder 5,
**dadurch gekennzeichnet,**
**dass** sie mindestens eine Kontrollfläche zum Nachweis von Störungen umfaßt, die durch eine nicht-analytspezifische Bindung von Komponenten des Nachweismediums an die Testfläche hervorgerufen werden.

7. Festphase nach einem der vorhergehenden Ansprüche 4 bis 6,
**dadurch gekennzeichnet,**
**dass** sie mindestens eine Kontrollfläche zum Nachweis von Störungen umfaßt, die durch eine nicht-analytspezifische Bindung von Komponenten des Nachweismediums an den Festphasenrezeptor auf einer Testfläche hervorgerufen werden.

8. Festphase nach Anspruch 6 oder 7,
**dadurch gekennzeichnet,**
**dass** sie mindestens eine Kontrollfläche zum Nachweis von Störungen umfaßt, die durch eine nicht-analytspezifische Bindung von Komponenten des Nachweismediums an immobilisierte Antikörper, Antikörperfragmente, Antigene oder/und Nukleinsäuren auf einer Testfläche hervorgerufen werden.

9. Festphase nach Anspruch 7 oder 8,
**dadurch gekennzeichnet,**
**daß** die Kontrollfläche einen modifizierten Festphasenrezeptor enthält, der sich von einem Festphasenrezeptor auf einer Testfläche durch das Fehlen einer analytspezifischen Bindungsstelle unterscheidet.

10. Festphase nach Anspruch 7, 8 oder 9,
**dadurch gekennzeichnet,**
**dass** sie mindestens eine Kontrollfläche zum Nachweis von Störungen durch Rheumafaktoren umfaßt.

11. Festphase nach Anspruch 7, 8 oder 9,
**dadurch gekennzeichnet,**
**dass** sie mindestens eine Kontrollfläche zum Nachweis von Störungen durch fremdspezies-spezifische Antikörper umfaßt.

12. Festphase nach einem der vorhergehenden Ansprüche 4 bis 11,
**dadurch gekennzeichnet,**
**dass** sie mindestens eine Kontrollfläche zum Nachweis von Störungen umfaßt, die durch die unspezifische Bindung von Komponenten des Nachweismediums an nicht-analytspezifische Substanzen auf dem Festphasenträger hervorgerufen werden.

13. Festphase nach Anspruch 12,
**dadurch gekennzeichnet,**
**dass** sie mindestens eine Streptavidin enthaltende Testfläche und mindestens eine Kontrollfläche zum Nachweis von Störungen durch nicht-analytspezifische Streptavidin-bindende Komponenten des Nachweismediums umfaßt.

14. Festphase nach Anspruch 6,
**dadurch gekennzeichnet,**
**dass** sie mindestens eine Kontrollfläche zum Nachweis des Gesamt-IgE-Gehalts umfaßt.

15. Festphase nach einem der Ansprüche 4 bis 14,
**dadurch gekennzeichnet,**
**daß** sie weiterhin mindestens eine Positiv-Referenzfläche mit dem zu bestimmenden Analyten umfaßt.

16. Festphase nach einem der Ansprüche 4 bis 15,
**dadurch gekennzeichnet,**
**daß** die Testflächen und Kontrollflächen jeweils einen Durchmesser von 10 µm bis 1 cm aufweisen.

17. Verwendung einer Festphase nach einem der Ansprüche 1 bis 16 zum Nachweis eines Analyten in einer Probe.

18. Verwendung nach Anspruch 17,
**dadurch gekennzeichnet,**
**daß** die Festphase mehrere Testflächen zum gleichzeitigen Nachweis mehrerer Analyten umfaßt.

19. Verwendung nach Anspruch 17 oder 18 in einem Immunoassay.

20. Verwendung nach Anspruch 17 oder 18 in einem Nukleinsäure-Hybridisierungsassay.

## Claims

1. Method for the detection of an analyte by specifically binding the analyte to a solid phase receptor in which interferences which are caused by unspecific binding of interfering non-analyte components to the test area are additionally detected using a solid phase having at least one defined test area which has a solid phase receptor with an analyte-specific receptor binding site, wherein the solid phase additionally comprises at least one defined control area for the detection of interferences which are caused by unspecific binding of interfering non-analyte components to the test area, wherein the solid phase receptor with the analyte-specific receptor binding site was applied using a loading solution and wherein the control area has components of the test area and namely reagents of the loading solution but not the analyte-specific receptor binding site.

2. Method according to claim 1,
**characterized in that**
the control areas are used for the quantitative correction of interferences.

3. Use of defined control areas to detect interferences which are caused by non-analyte components in a method for the detection of an analyte by specific binding of the analyte to an analyte-specific receptor binding site of a solid phase receptor in a defined test area, wherein the solid phase receptor with the analyte-specific receptor binding site was applied using a loading solution and wherein the control area has components of the test area and namely reagents of the loading solution but not the analyte-specific receptor binding site.

4. Solid phase with at least one defined test area comprising a solid phase receptor for the detection of an analyte in a sample by specific binding of the analyte to an analyte-specific receptor binding site of the solid phase receptor,
**characterized in that**
it additionally comprises at least one defined control area to detect interferences which are caused by unspecific binding of interfering non-analyte components to the test area, wherein the solid phase receptor with the analyte-specific receptor binding site was applied using a loading solution and wherein the control area has components of the test area and namely reagents of the loading solution but not the analyte-specific receptor binding site.

5. Solid phase according to claim 4,
**characterized in that**
it comprises several test areas for the simultaneous detection of several analytes in a sample.

6. Solid phase according to one of the claims 4 or 5,
**characterized in that**
it comprises at least one control area to detect interferences which are caused by a non-analyte specific binding of components of the detection medium to the test area.

7. Solid phase according to one of the previous claims 4 to 6,
**characterized in that**
it comprises at least one control area to detect interferences which are caused by a non-analyte specific binding of components of the detection medium to the solid phase receptor on a test area.

8. Solid phase according to claim 6 or 7,
**characterized in that**
it comprises at least one control area to detect interferences which are caused by a non-analyte specific binding of components of the detection medium to immobilized antibodies, antibody fragments, antigens or/and nucleic acids on a test area.

9. Solid phase according to claim 7 or 8,
**characterized in that**
the control area contains a modified solid phase receptor which differs from a solid phase receptor on a test area by the absence of an analyte-specific binding site.

10. Solid phase according to claim 7, 8 or 9,
**characterized in that**
it comprises at least one control area to detect interferences by rheumatoid factors.

11. Solid phase according to claim 7, 8 or 9,
**characterized in that**
it comprises at least one control area to detect interferences by foreign-species-specific antibodies.

12. Solid phase according to one of the previous claims 4 to 11,
**characterized in that**
it comprises at least one control area to detect interferences which are caused by unspecific binding of components of the detection medium to non-analyte specific substances on the solid phase support.

13. Solid phase according to claim 12,
**characterized in that**
it comprises at least one test area containing streptavidin and at least one control area to detect interferences by non-analyte specific streptavidin binding components of the detection medium.

14. Solid phase according to claim 6,
**characterized in that**
it comprises at least one control area to detect the total IgE content.

15. Solid phase according to one of the claims 4 to 14,
**characterized in that**
it additionally comprises at least one positive reference area containing the analyte to be determined.

16. Solid phase according to one of the claims 4 to 15,
**characterized in that**
the test areas and control areas each have a diameter of 10 µm to 1 cm.

17. Use of a solid phase according to one of the claims 1 to 16 to detect an analyte in a sample.

18. Use according to claim 17,
**characterized in that**
the solid phase comprises several test areas for the simultaneous detection of several analytes.

19. Use according to claim 17 or 18 in an immunoassay.

20. Use according to claim 17 or 18 in a nucleic acid hybridization assay.

## Revendications

1. Procédé de détection d'un analyte par liaison spécifique de l'analyte à un récepteur en phase solide, des perturbations dues à des liaisons non spécifiques de particules parasites étrangères à l'analyte sur la surface de test étant en outre détectées, en utilisant une phase solide comportant au moins une surface de test limitée qui présente un récepteur en phase solide avec un site de liaison au récepteur spécifique à l'analyte, la phase solide comprenant en outre au moins une surface témoin limitée pour détecter des perturbations qui sont dues à des liaisons non spécifiques de particules parasites étrangères à l'analyte sur la surface de test, le récepteur en phase solide comportant le site de liaison au récepteur spécifique à l'analyte étant appliqué en utilisant une solution de charge et la surface témoin présentant des composants de la surface de test, à savoir des réactifs de la solution de charge, mais non le site de liaison au récepteur spécifique de l'analyte.

2. Procédé selon la revendication 1,
**caractérisé en ce**
**que** la surface témoin est utilisée pour la correction quantitative de perturbations.

3. Utilisation de surfaces témoins limitées pour identifier des perturbations qui sont dues à des composants étrangers à l'analyte, dans un procédé de détection d'un analyte par liaison spécifique de l'analyte à un site de liaison au récepteur spécifique de l'analyte, d'un récepteur en phase solide dans une surface de test limitée, le récepteur en phase solide comportant le site de liaison au récepteur spécifique de l'analyte étant appliqué en utilisant une solution de charge et la surface témoin présentant des composants de la surface de test, à savoir des réactifs de la solution de charge, mais non le site de liaison au récepteur spécifique de l'analyte.

4. Phase solide comportant au moins une surface de test limitée, comprenant un récepteur en phase solide, pour la détection d'un analyte dans un échantillon par liaison spécifique de l'analyte à un site de liaison au récepteur spécifique de l'analyte du récepteur en phase solide,
**caractérisée en ce**
**qu'**elle présente en outre au moins une surface témoin limitée pour la détection de perturbations qui sont dues à des liaisons non spécifiques de particules parasites étrangères à l'analyte sur la surface de test, le récepteur en phase solide comportant le site de liaison au récepteur spécifique de l'analyte étant appliqué en utilisant une solution de charge et la surface témoin présentant des composants de la surface de test, à savoir des réactifs de la solution de charge, mais non le site de liaison au récepteur spécifique de l'analyte.

5. Phase solide selon la revendication 4,
**caractérisée en ce**
**qu'**elle comprend plusieurs surfaces de test pour la détection concomitante de plusieurs analytes dans un échantillon.

6. Phase solide selon l'une des revendications 4 ou 5,
**caractérisée en ce**
**qu'**elle comprend au moins une surface témoin pour la détection de perturbations qui sont dues à une liaison non spécifique à l'analyte de composants du milieu de détection sur la surface de test.

7. Phase solide selon l'une des revendications précédentes 4 à 6,
**caractérisée en ce**
**qu'**elle comprend au moins une surface témoin pour la détection de perturbations qui sont dues à une liaison non spécifique à l'analyte de composants du milieu de détection au récepteur en phase solide sur une surface de test.

8. Phase solide selon la revendication 6 ou 7,
**caractérisée en ce**
**qu'**elle comprend au moins une surface témoin pour la détection de perturbations qui sont dues à une liaison non spécifique à l'analyte de composants du milieu de détection avec des anticorps immobilisés, des fragments d'anticorps, des antigènes et/ou des acides nucléiques sur une surface de test.

9. Phase solide selon la revendication 7 ou 8,
**caractérisée en ce**
**que** la surface témoin contient un récepteur en phase solide modifié qui se distingue d'un récepteur en phase solide sur une surface de test par l'absence d'un site de liaison spécifique à l'analyte.

10. Phase solide selon la revendication 7, 8 ou 9,
**caractérisée en ce**
**qu'**elle comprend au moins une surface témoin pour la détection de perturbations par des facteurs rhumatismaux.

11. Phase solide selon la revendication 7, 8 ou 9,
**caractérisée en ce**
**qu'**elle comprend au moins une surface témoin pour la détection de perturbations par des anticorps spécifiques d'une espèce étrangère.

12. Phase solide selon l'une quelconque des revendications 4 à 11,
**caractérisée en ce**
**qu'**elle comprend au moins une surface témoin pour la détection de perturbations qui sont dues à une liaison non spécifique de composants du milieu de détection avec des substances non spécifiques de l'analyte sur le support en phase solide.

13. Phase solide selon la revendication 12,
**caractérisée en ce**
**qu'**elle comprend au moins une surface de test contenant de la streptavidine et au moins une surface témoin pour la détection de perturbations dues à des composants du milieu de détection non spécifiques à l'analyte se liant à la streptavidine.

14. Phase solide selon la revendication 6,
**caractérisée en ce**
**qu'**elle comprend au moins une surface témoin pour la détection de la teneur en IgE totale.

15. Phase solide selon l'une des revendications 4 à 14,
**caractérisée en ce**
**qu'**elle comprend en outre au moins une surface de référence positive, avec l'analyte à déterminer.

16. Phase solide selon l'une des revendications 4 à 15,
**caractérisée en ce**
**que** les surfaces de test et les surfaces témoins présentent chacune un diamètre de 10 µm à 1 cm.

17. Utilisation d'une phase solide selon l'une des revendications 1 à 16, pour la détection d'un analyte dans un échantillon.

18. Utilisation selon la revendication 17,
**caractérisée en ce**
**que** la phase solide comprend plusieurs surfaces de test pour la détection concomitante de plusieurs analytes.

19. Utilisation selon la revendication 17 ou 18 dans un essai immunologique.

20. Utilisation selon la revendication 17 ou 18, dans un essai d'hybridation d'acide nucléique.
